# EUROPEAN PATENT SPECIFICATION

(11) **EP 2 952 149 B1**
(45) Date of publication and mention of the grant of the patent: **20.02.2019**
(21) Application number: 13873951.1
(22) Date of filing: 09.12.2013
(51) Int. Cl.: A61B 18/04, A61B 18/08, A61B 18/14, A61B 18/00

(54) **THERAPEUTIC TREATMENT DEVICE**
THERAPEUTISCHE BEHANDLUNGSVORRICHTUNG
DISPOSITIF DE TRAITEMENT THÉRAPEUTIQUE

(30) Priority: 30.01.2013 JP 2013015343
(43) Date of publication of application: 09.12.2015
(73) Proprietor: Olympus Corporation, Tokyo 192-8507 (JP)
(72) Inventor: NAKAMURA, Kotaro, Hachioji-shi, Tokyo 192-8507 (JP)
(74) Representative: Gunzelmann, Rainer
(86) International application number: PCT/JP2013/082976
(87) International publication number: WO 2014/119137

(56) References cited:
- EP-A1- 1 532 933
- WO-A1-2012/043469
- WO-A1-2012/161163
- JP-A- 2005 348 820
- JP-A- 2011 218 182

## Description

### Technical Field

The present invention relates to a therapeutic treatment apparatus.

### Background Art

There is generally known a therapeutic treatment apparatus for treating body tissues by use of thermal energy. For example, Japanese Patent Application Laid-Open No. 2006-305236 Publication discloses therein a therapeutic treatment apparatus described later. That is, in the therapeutic treatment apparatus, a substrate for heat generation element is made of a metal member with excellent thermal conductivity. The substrate is formed with a heat generation part having a thin film resistance. The heat generation part is joined with a lead wire for supplying power. A filling agent is provided around a portion where the heat generation part and the lead wire are joined with each other, thereby securing an electric insulation property. The substrate for heat generation element has high thermal conductivity such that even if a body tissue partially contacts on such a heat generation element, preferable temperature controllability is secured. Therefore, the substrate is formed to be relatively thick by use of a member with higher thermal conductivity.
Further, document EP 1 532 933 A1 describes an over shoe for use with electrosurgical instruments having a pair of juxtaposed jaw members pivotably associated with one another. The over shoe includes a tissue contacting wall and a pair of side walls terminating a bottom wall, wherein the walls of the over shoe define a cavity therein sized and dimensioned to receive one of the pair of opposing jaw members. In use, electrosurgical energy flows from the positive terminal of an electrosurgical generator to an electrode pad of a jaw member through an aperture of the over shoe placed on the jaw member, through tissue, through a further aperture of a further over shoe placed on the opposite jaw member, and back to the negative terminal of the electrosurgical generator.
In document WO 2012/043469 A1, a therapeutic treatment system is described that includes a pair of retaining members, wherein at least one of the retaining members moves relatively with respect to the other to retain biological tissue, and a plurality of heater members that are provided on at least one of the retaining members and that are arranged dispersedly and separately from one another.
Document WO 2012/161163 A1 discloses a therapeutic treatment apparatus for treatment by heating body tissue to a target temperature is provided with heat transfer parts transferring heat to the body tissue, a heat-generating tip having a heat-generating member, first temperature-sensing parts, a temperature-sensing element disposed on the heat transfer parts, a second temperature-sensing part, and a controller.

### Summary of Invention

For example, in the therapeutic treatment apparatus according to Japanese Patent Application Laid-Open No. 2006-305236 Publication described above, the heat generation part is provided to be stacked in the thickness direction of the heat generation element on the backside opposite to the surface of the substrate contacting with a body tissue, and the lead wire connected to the heat generation part is provided to be further stacked in the thickness direction of the heat generation element. On the other hand, in the therapeutic treatment apparatus, thinning or downsizing in height of the heat generation element is required.

It is therefore an object of the present invention to provide a thinned therapeutic treatment apparatus.

The present invention concerns a therapeutic treatment apparatus according to claim 1.
According to one aspect of the present invention, a therapeutic treatment apparatus is
directed for heating and treating a body tissue, the apparatus comprising a heat transfer plate configured to contact with the body tissue on a first main surface out of the first main surface and a second main surface which are the front and back surfaces, and to transfer heat to the body tissue, an electrothermal conversion element which includes extension parts provided on the second main surface of the heat transfer plate and extending from the heat transfer plate and is formed with an electric resistance pattern for generating heat in response to an applied voltage and first lead connection parts connected to the electric resistance pattern and provided on the extension parts, fixing parts provided at ends of the heat transfer plate on the side where the electrothermal conversion element extends, and directed to sandwich the electrothermal conversion element between them and the heat transfer plate, and first lead wires electrically connected to the first lead connection parts at the extension parts and directed to supply the electric resistance pattern with power.

According to the present invention, the lead wires for supplying power are connected to the extension parts extending from the heat transfer plate, thereby providing a thinned therapeutic treatment apparatus.

### Brief Description of Drawings

Fig. 1 is a schematic diagram illustrating an exemplary structure of a therapeutic treatment system according to each exemplary embodiment;
Fig. 2A is a schematic cross-section view illustrating an exemplary structure of a shaft and a holding part in an energy treatment tool according to each exemplary embodiment, which illustrates a state in which the holding part is closed;
Fig. 2B is a schematic cross-section view illustrating an exemplary structure of the shaft and the holding part in the energy treatment tool according to each exemplary embodiment, which illustrates a state in which the holding part is opened;
Fig. 3A is a schematic plan view illustrating an exemplary structure of a first holding member in the holding part according to each exemplary embodiment;
Fig. 3B is a schematic diagram illustrating an exemplary structure of the first holding member in the holding part according to each exemplary embodiment, which is a longitudinal cross-section view along the line 3B-3B illustrated in Fig. 3A;
Fig. 3C is a schematic diagram illustrating an exemplary structure of the first holding member in the holding part according to each exemplary embodiment, which is a transverse cross-section view along the line 3C-3C illustrated in Fig. 3A;
Fig. 4 is an exploded perspective view illustrating an exemplary structure of a first electrode part according to a first exemplary embodiment;
Fig. 5 is a perspective view illustrating an exemplary structure of a first high frequency electrode according to the first exemplary embodiment;
Fig. 6 is an enlarged perspective view illustrating an exemplary structure of a base end of the first high frequency electrode according to the first exemplary embodiment;
Fig. 7 is a plan view illustrating an exemplary structure of an electrothermal conversion element according to the first exemplary embodiment;
Fig. 8 is a plan view illustrating an exemplary structure of the first high frequency electrode according to the first exemplary embodiment;
Fig. 9 is a side view illustrating an exemplary structure of the first high frequency electrode according to the first exemplary embodiment;
Fig. 10 is a side view illustrating an exemplary structure of the base end of the first high frequency electrode according to the first exemplary embodiment;
Fig. 11 is a perspective view illustrating an exemplary structure of part of the first electrode part according to the first exemplary embodiment;
Fig. 12 is a perspective view illustrating an exemplary structure of part of the first electrode part according to the first exemplary embodiment;
Fig. 13 is a perspective view illustrating an exemplary structure of the first electrode part according to the first exemplary embodiment;
Fig. 14 is a perspective view illustrating an exemplary state in which first heater current lines and a first high frequency electrode current line are connected to part of the first electrode part according to the first exemplary embodiment;
Fig. 15 is a perspective view illustrating an exemplary structure of the first high frequency electrode according to a second exemplary embodiment; and
Fig. 16 is a perspective view illustrating an exemplary structure of part of the first electrode part according to the second exemplary embodiment.

### Description of Embodiments

### [First exemplary embodiment]

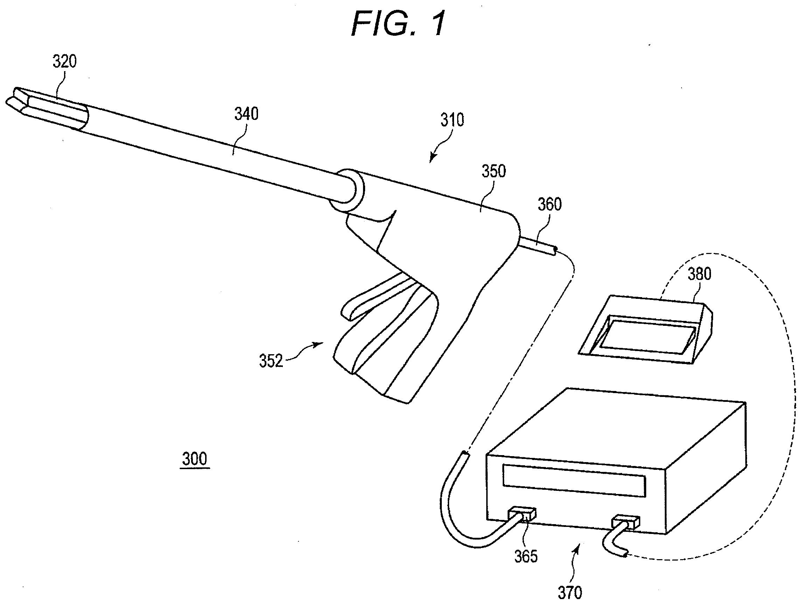

A first exemplary embodiment according to the present invention will be described with reference to the drawings. A therapeutic treatment apparatus according to the present exemplary embodiment is used for treating body tissues. The therapeutic treatment apparatus operates high frequency energy and thermal energy on body tissues. An appearance of a therapeutic treatment apparatus 300 is schematically illustrated in Fig. 1. As illustrated, the therapeutic treatment apparatus 300 comprises an energy treatment tool 310, a control device 370, and a foot switch 380.

The energy treatment tool 310 is a linear type surgical treatment tool penetrating through the abdominal wall for treatment, for example. The energy treatment tool 310 includes a handle 350, a shaft 340 attached on the handle 350, and a holding part 320 provided on the tip end of the shaft 340. The holding part 320 is a treatment part which is openable/closable and is directed to do treatments such as coagulation and incision of a body tissue by gripping the body tissue to be treated. For the following description, the side of the holding part 320 will be called tip end side and the side of the handle 350 will be called base end side. The handle 350 comprises a plurality of operation knobs 352 for operating the holding part 320. The handle 350 is further provided with a non-volatile memory (not illustrated) for storing therein eigenvalues and the like for the energy treatment tool 310. A shape of the energy treatment tool 310 illustrated herein is exemplary, and any other shape having the same function may be employed. For example, a forceps-like shape may be employed and the shaft may be curved.

The handle 350 is connected to the control device 370 via a cable 360. Herein, the cable 360 and the control device 370 are connected with each other via a connector 365, and the connection is detachable. That is, the therapeutic treatment apparatus 300 is configured to replace the energy treatment tool 310 per treatment. The control device 370 is connected with the foot switch 380. The foot-operated foot switch 380 may be replaced with a hand-operated switch or other switch. An operator operates the pedal of the foot switch 380 thereby to switch ON/OFF energy supply from the control device 370 to the energy treatment tool 310.

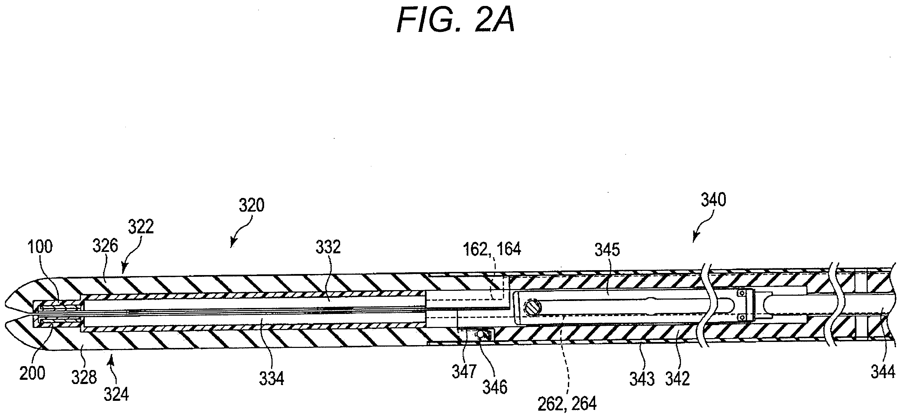

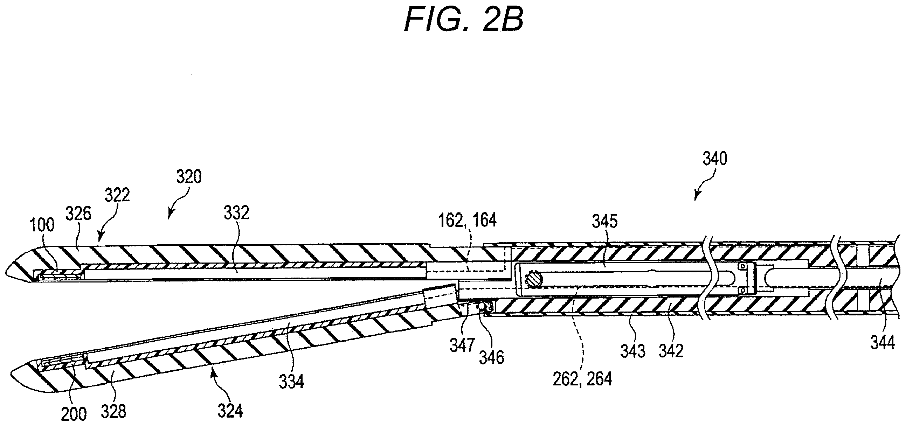

An exemplary structure of the holding part 320 and the shaft 340 is illustrated in Fig. 2A and Fig. 2B. Fig. 2A illustrates a state in which the holding part 320 is closed and Fig. 2B illustrates a state in which the holding part 320 is opened. The shaft 340 comprises a tube 342 and a sheath 343. The tube 342 is fixed at its base end to the handle 350. The sheath 343 is slidably arranged on the outer periphery of the tube 342 in the axial direction of the tube 342.

The holding part 320 is arranged on the tip end of the tube 342. The holding part 320 comprises a first holding member 322 and a second holding member 324. The base of the first holding member 322 is fixed on the tip end of the tube 342 in the shaft 340. On the other hand, the base of the second holding member 324 is rotatably supported on the tip end of the tube 342 in the shaft 340 by a support pin 346. Therefore, the second holding member 324 axially rotates about the support pin 346 and opens/closes relative to the first holding member 322.

In a state in which the holding part 320 is closed, a cross-section shape in which the base of the first holding member 322 and the base of the second holding member 324 are put together is circular. The second holding member 324 is energized by an elastic member 347 such as plate spring to open relative to the first holding member 322. When the sheath 343 is slid toward the tip end of the tube 342 so that the base of the first holding member 322 and the base of the second holding member 324 are covered by the sheath 343, as illustrated in Fig. 2A, the first holding member 322 and the second holding member 324 are closed against an energizing force of the elastic member 347. On the other hand, when the sheath 343 is slid toward the base end side of the tube 342, as illustrated in Fig. 2B, the second holding member 324 is opened relative to the first holding member 322 due to an energizing force of the elastic member 347.

The tube 342 is inserted with a first high frequency electrode current line 162 connected to a first high frequency electrode 110 and a second high frequency electrode current line 262 connected to a second high frequency electrode 210, which will be described later. The tube 342 is inserted with a pair of first heater current lines 164 connected to an electrothermal conversion element 140 as a heat generation member described later arranged on the first high frequency electrode 110 and a pair of second heater current lines 264 connected to an electrothermal conversion element 230 arranged on a second high frequency electrode 210.

A drive rod 344 connected on its base end to one of the operation knobs 352 is movably arranged in the axial direction of the tube 342 inside the tube 342. A sheet-shaped cutter 345 forming a blade on its tip end is arranged on the tip end of the drive rod 344. When the operation knob 352 is operated, the cutter 345 is moved in the axial direction of the tube 342 via the drive rod 344. When the cutter 345 is moved toward the tip end, the cutter 345 is housed in a first cutter guide groove 332 and a second cutter guide groove 334 described later formed in the holding part 320.

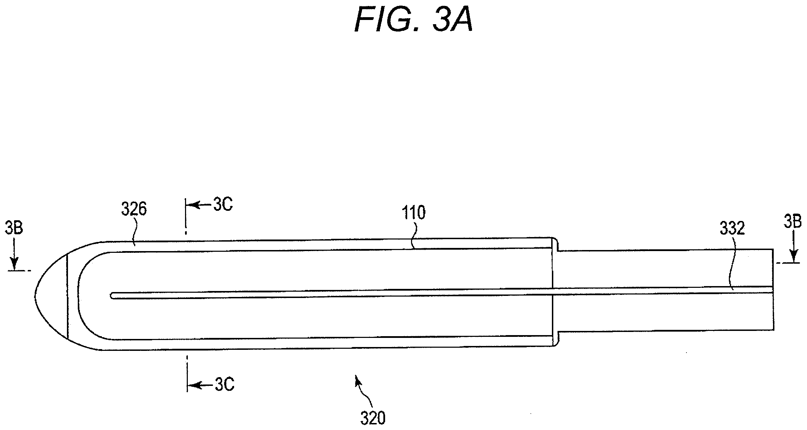

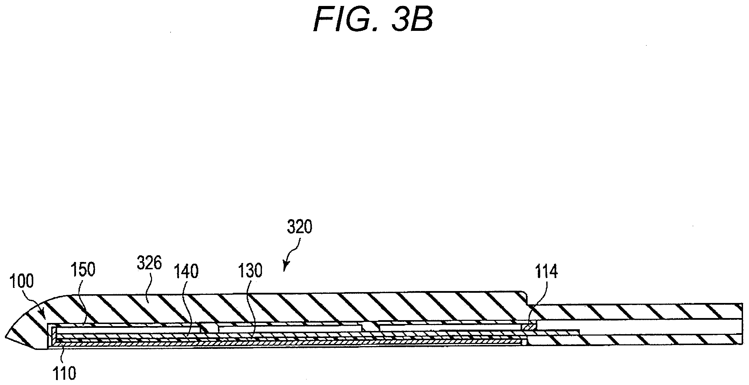

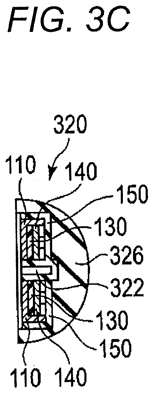

A structure of the first holding member 322 is schematically illustrated in Fig. 3A, Fig. 3B and Fig. 3C. As illustrated, the first holding member 322 is formed with the first cutter guide groove 332 for guiding the cutter 345. The first holding member 322 is provided with the first high frequency electrode 110 including a copper thin plate, for example. The first high frequency electrode 110 is configured to contact with a body tissue on either main surface thereof (which will be called first main surface below) . The first high frequency electrode 110 includes the first cutter guide groove 332, and thus its planar shape is U-shaped as illustrated in Fig. 3A. The first high frequency electrode 110 is electrically connected with the first high frequency electrode current line 162 as described later in detail. The first high frequency electrode 110 is connected to the control device 370 via the first high frequency electrode current line 162 and the cable 360. The electrothermal conversion element 140 and a cover member 150 are arranged on a second main surface of the first high frequency electrode 110 which does not contact with a body tissue as described later in detail. A first electrode part 100 formed of the first high frequency electrode 110, the electrothermal conversion element 140, the cover member 150 and the like is formed in this way. The first electrode part 100 is embedded in and fixed on a first holding member main body 326. An exemplary structure of the first electrode part 100 will be described below in more detail.

As illustrated in Fig. 2A and Fig. 2B, the second holding member 324 is symmetrical in its shape to the first holding member 322, and has the same structure as the first holding member 322. That is, the second holding member 324 is formed with the second cutter guide groove 334 opposite to the first cutter guide groove 332. The second holding member 324 is provided with the second high frequency electrode 210 opposite to the first high frequency electrode 110. The second high frequency electrode 210 is configured to contact with a body tissue on either main surface thereof. The second high frequency electrode 210 is connected to the control device 370 via the second high frequency electrode current line 262 and the cable 360.

The electrothermal conversion element 230 and a cover member 250 are arranged on a surface of the second high frequency electrode 210 which does not contact with a body tissue. A second electrode part 200 formed of the second high frequency electrode 210, the electrothermal conversion element 230, the cover member 250 and the like is formed in this way. The second electrode part 200 is embedded in and fixed on a second holding member main body 328.

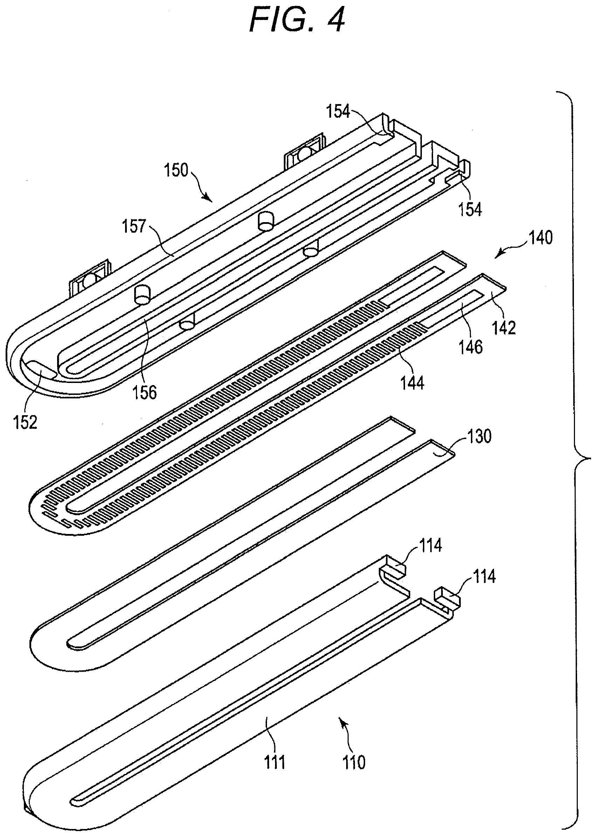

The first electrode part 100 will be described in detail. The second electrode part 200 has the same structure as the first electrode part 100, and thus the description of the second electrode part 200 will be omitted. An exploded perspective view of the first electrode part 100 is illustrated in Fig. 4. As illustrated, the first electrode part 100 includes the first high frequency electrode 110, a highly heat-conductive and heat-resistant adhesive sheet 130, the electrothermal conversion element 140, and the cover member 150. The first high frequency electrode 110, the highly heat-conductive and heat-resistant adhesive sheet 130, and the electrothermal conversion element 140 have a U shape to form the first cutter guide groove 332. The cover member 150 has a groove to form the first cutter guide groove 332.

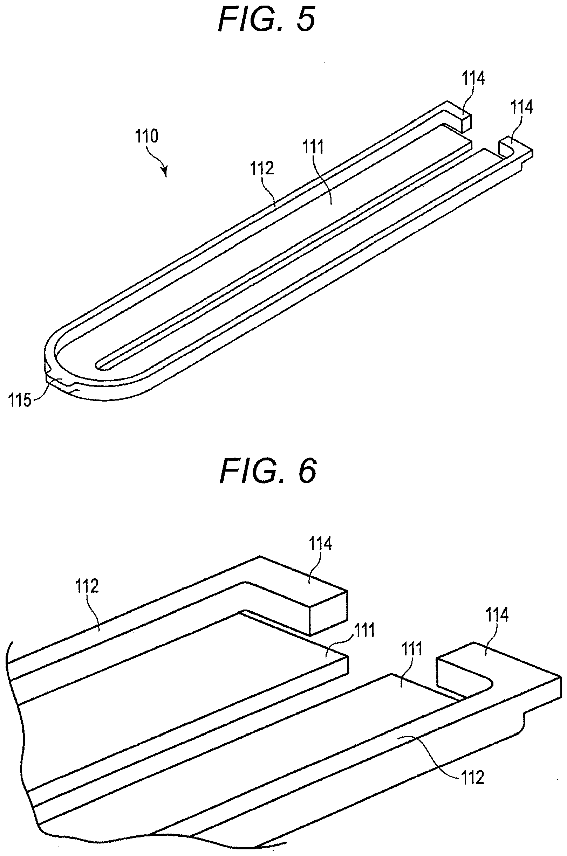

The first high frequency electrode 110 will be described with reference to Fig. 5 and Fig. 6. Fig. 5 is a perspective view of the first high frequency electrode 110, and Fig. 6 is an enlarged perspective view of the base end of the first high frequency electrode 110. The first high frequency electrode 110 is made of copper, for example, as described above. The first high frequency electrode 110 includes an electrode bottom 111 capable of contacting with a body tissue. A thickness of the electrode bottom 111 is around 0.5 mm, for example. A sidewall 112 is provided on the periphery of the electrode bottom 111 except the base end. The sidewall 112 is formed on the side of the cover member 150 to be perpendicular to the electrode bottom 111. Fixing parts 114 protruding from the sidewall 112 in the center axis direction of the first high frequency electrode 110 are provided at the base end of the first high frequency electrode 110. A convex part 115 protruding from the sidewall 112 is provided on the tip end of the first high frequency electrode 110.

The first high frequency electrode 110 may be formed by cutting work, for example. Further, the additionally-formed fixing parts 114 may be added to the sidewall 112 after the electrode bottom 111 and the sidewall 112 are formed. In this case, the electrode bottom 111 and the sidewall 112 may be formed also by bending work.

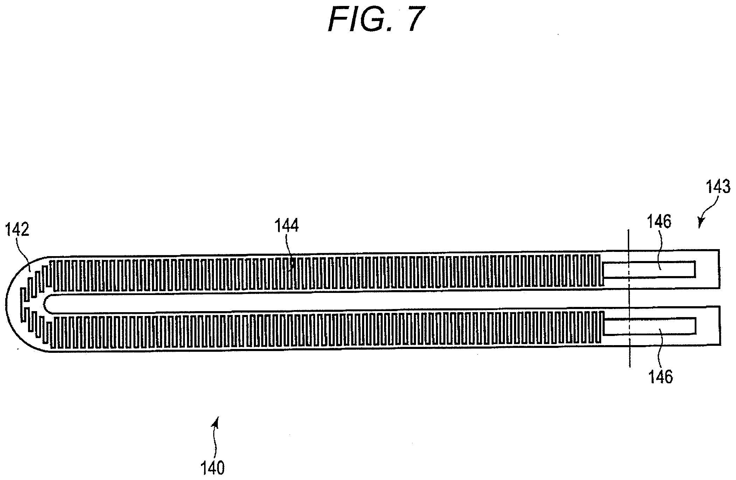

A plan view of the electrothermal conversion element 140 is illustrated in Fig. 7. As illustrated, the electrothermal conversion element 140 includes a substrate 142 made of polyimide, for example. The shape of the substrate 142 generally matches with the shape of the electrode bottom 111 of the first high frequency electrode 110, and the length thereof is slightly larger than that of the electrode bottom 111. A position corresponding to the end on the base end side of the electrode bottom 111 when the first electrode part 100 is assembled is indicated in a dashed-dotted line in Fig. 7. A portion protruding from the electrode bottom 111 will be called extension part 143.

An electric resistance pattern 144 is formed of a stainless (SUS) pattern, for example, in most of the substrate 142 except the extension parts 143. First lead connection parts 146 connected to both ends of the electric resistance pattern 144 are formed of a SUS pattern at the ends including the extension parts 143 of the substrate 142. When a voltage is applied to a pair of first lead connection parts 146, the electric resistance pattern 144 generates heat. In this way, the electrothermal conversion element 140 functions as a sheet heater. A thickness of the electrothermal conversion element 140 is around 100 µm, for example.

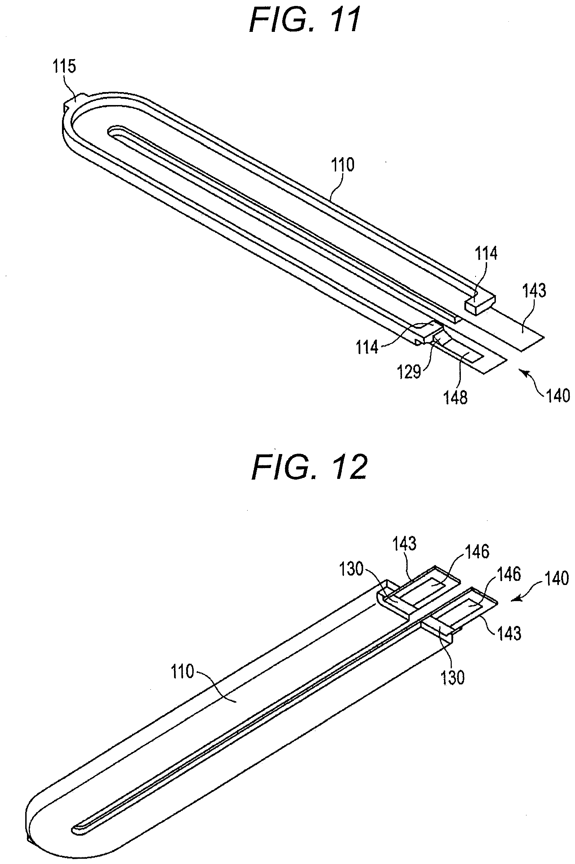

A second lead connection part 148 is formed on a main surface opposite to the main surface of the substrate 142 on which the electric resistance pattern 144 is formed as illustrated in Fig. 11 described later. The second lead connection part 148 is configured to contact with the fixing parts 114 of the first high frequency electrode 110. The second lead connection part 148 and the fixing part 114 are contacted with each other so that a voltage may be applied to the first high frequency electrode 110 via the second lead connection part 148.

The electrode bottom 111 and the electrothermal conversion element 140 in the first high frequency electrode 110 are adhered to each other by the highly high-conducive and high-resistant adhesive sheet 130. Herein, the electrothermal conversion element 140 is adhered with the surface forming the electric resistance pattern 144 thereon faced toward the first high frequency electrode 110. The highly high-conductive and high-resistant adhesive sheet 130 is a sheet which is high in thermal conductivity and resistant to high temperature and has an adhesive property. The highly heat-conductive and heat-resistant adhesive sheet 130 is made by mixing highly heat-conductive ceramic such as alumina or aluminum nitride with epoxy resin, for example. The highly heat-conductive and heat-resistant adhesive sheet 130 has a high adhesive performance, preferable thermal conductivity and an electric insulation property. A thickness of the highly heat-conductive and heat-resistant adhesive sheet 130 is around 50 µm, for example.

The highly heat-conductive and heat-resistant adhesive sheet 130 has substantially the same shape as the electrode bottom 111. The highly heat-conductive and heat-resistant adhesive sheet 130 is slightly longer than the electrode bottom 111 of the first high frequency electrode 110. Since the highly heat-conductive and heat-resistant adhesive sheet 130 is longer than the electrode bottom 111, an electric insulation property between the first high frequency electrode 110 and the first lead connection parts 146 is secured.

The cover member 150 is made of heat-resistant resin. The cover member 150 has a shape corresponding to the first high frequency electrode 110. A thickness of the cover member 150 is about 0.3 mm, for example. As illustrated in Fig. 4, a cutout 152 is provided on the tip end of the cover member 150. The cutout 152 engages with the convex part 115 of the first high frequency electrode 110. Further, convex parts 154 are provided on the base end side of the cover member 150. The convex parts 154 engage with the fixing parts 114 of the first high frequency electrode 110. In this way, the first high frequency electrode 110 and the cover member 150 are joined to each other via the engagements between the convex part 115 and the fixing parts 114 in the first high frequency electrode 110, and the cutout 152 and the convex parts 154 in the cover member 150. In this way, the first high frequency electrode 110 and the cover member 150 are joined to each other in a simple structure, thereby restricting manufacture cost. The cover member is provided with an inner wall 156 for forming the first cutter guide groove 332 and an outer wall 157 for covering the sidewall 112 of the first high frequency electrode 110.

In the structure of the first electrode part 100, the thickness of the first high frequency electrode 110 is relatively larger than those of other members. This is because thermal conductivity of the first high frequency electrode 110 is increased thereby to make a temperature of the first high frequency electrode 110 uniform even if a body tissue partially contacts on the first high frequency electrode 110. This is important in temperature control when a body tissue is anastomosed or joined by the energy treatment tool 310 according to the present exemplary embodiment.

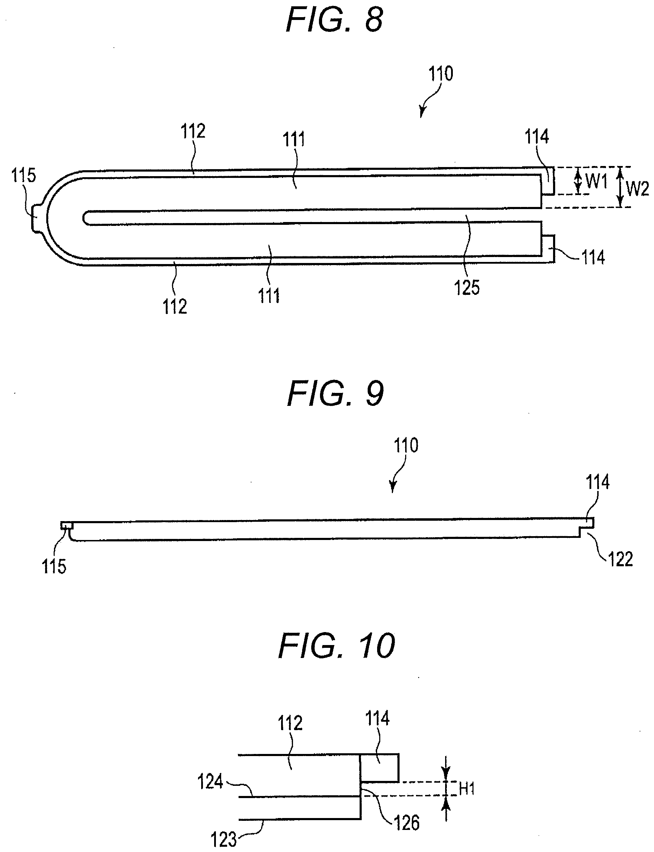

The first high frequency electrode 110 will be further described with reference to Fig. 8 to Fig. 10. Fig. 8 is a top view of the first high frequency electrode 110, Fig. 9 is a side view of the first high frequency electrode 110, and Fig. 10 is an enlarged side view of the first high frequency electrode 110 viewed from the inside of the sidewall 112. As illustrated in Fig. 8, the end faces on the tip end side of the fixing parts 114 and the end face on the base end side of the electrode bottom 111 are arranged on the same plane. The fixing parts 114 may be provided at the ends on the base end side of the electrode bottom 111, and thus the end faces on the tip end side of the fixing parts 114 may be arranged closer to the base end side than to the end face on the base end side of the electrode bottom 111. That is, as viewed from above, a gap may be present between the electrode bottom 111 and the fixing parts 114. Further, for example, the end faces on the tip end side of the fixing parts 114 may be arranged closer to the tip end side than to the end face on the base end side of the electrode bottom 111. That is, as viewed from above, the electrode bottom 111 and the fixing parts 114 may be arranged to be overlapped on each other.

As illustrated in Fig. 8, in terms of either side across the cutout 125 forming the first cutter guide groove 332, it is preferable that a width W1 of the fixing part 114 is larger than half of a width W2 of the first high frequency electrode 110. That is, W1 > W2/2 is preferable. This is because the fixing parts 114 sufficiently restricts the electrothermal conversion element 140 as described later. The width W1 of the fixing part 114 may be changed depending on hardness of the electrothermal conversion element 140 as needed.

As illustrated in Fig. 9, concave parts 122 are provided on the electrode bottom 111 side of the fixing parts 114. The concave parts 122 engage with the convex parts 154 of the cover member 150.

As illustrated in Fig. 10, a surface contacting with a body tissue, out of the main surfaces of the electrode bottom 111, will be called first main surface 123, and a face forming the sidewall 112 thereon will be called second main surface 124. A height HI of a gap 126 between the surface on the electrode bottom 111 side of the fixing part 114 and the second main surface 124 substantially matches with a total thickness of the highly heat-conductive and heat-resistant adhesive sheet 130 and the electrothermal conversion element 140. The height of the gap 126 is around 150 µm, for example.

The second main surface of the electrode bottom 111 is attached with the electrothermal conversion element 140 by the highly heat-conductive and heat-resistant adhesive sheet 130. Herein, the electrothermal conversion element 140 is arranged with the surface forming the electric resistance pattern 144 thereon faced toward the electrode bottom 111 as illustrated in Fig. 4. The base end of the electrothermal conversion element 140 is inserted into the gap 126 between the electrode bottom 111 and the fixing parts 114.

The perspective views where the electrothermal conversion element 140 is arranged in the first high frequency electrode 110 are illustrated in Fig. 11 and Fig. 12. As illustrated, the extension parts 143 of the electrothermal conversion element 140 protrude from the base end of the first high frequency electrode 110. As illustrated in Fig. 11, the second lead connection part 148 provided on the extension part 143 contacts with the fixing part 114 of the first high frequency electrode 110. The second lead connection part 148 and the fixing part 114 are fixed by conductive paste 129 so that electric conduction therebetween is secured. Connection by welding or soldering, for example, may be employed instead of the connection by the conductive paste 129. As illustrated in Fig. 12, the highly heat-conductive and heat-resistant adhesive sheet 130 protrudes from the electrode bottom 111 thereby to secure insulation between the first high frequency electrode 110 and the first lead connection parts 146.

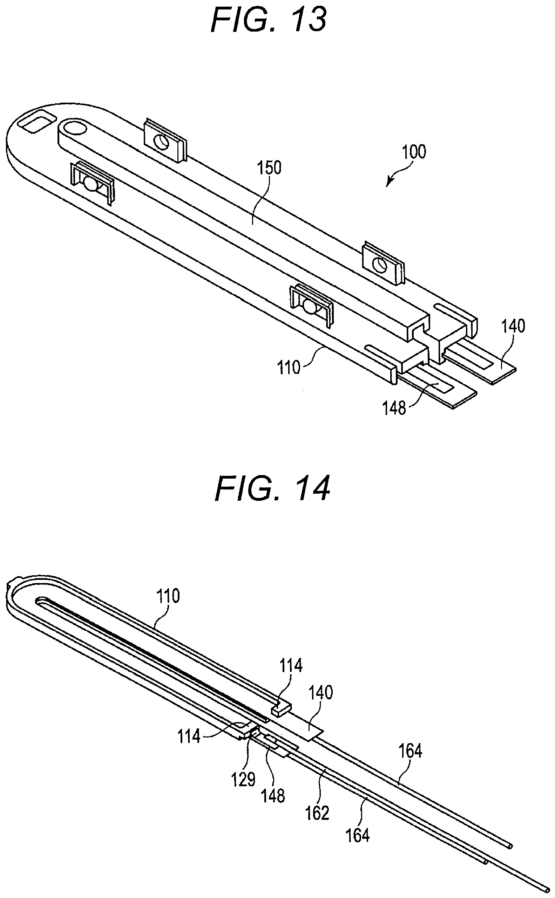

The first high frequency electrode 110 attached with the electrothermal conversion element 140 is fit with the cover member 150 as illustrated in Fig. 13. The first electrode part 100 is formed in this way.

As illustrated in Fig. 14, the second lead connection part 148 is connected with the first high frequency electrode current line 162 by soldering, for example. A high frequency voltage is applied to the first high frequency electrode 110 from the first high frequency electrode current line 162 via the second lead connection part 148 so that the first high frequency electrode 110 applies a high frequency current to a body tissue gripped by the holding part 320. The first lead connection parts 146 are connected with the first heater current lines 164 by soldering, for example. A voltage is applied to the electric resistance pattern 144 from the first heater current lines 164 via the first lead connection parts 146 so that the electric resistance pattern 144 generates heat and the heat is transferred to the body tissue via the first high frequency electrode 110.

When the second lead connection part 148 and the first high frequency electrode current line 162 are connected with each other and the first lead connection parts 146 and the first heater current lines 164 are connected with each other, the connection portions therebetween are preferably applied with a sealing agent made of silicon resin (not illustrated), for example.

The electric resistance pattern 144 of the electrothermal conversion element 140 is arranged closer to the first high frequency electrode 110 than to the substrate 142 of the electrothermal conversion element 140 with the highly heat-conductive and heat-resistant adhesive sheet 130 intervened between the electric resistance pattern 144 and the first high frequency electrode 110. Thus, the electric resistance pattern 144 is thermally coupled with the first high frequency electrode 110 via the highly heat-conductive and heat-resistant adhesive sheet 130. Only the highly heat-conductive and heat-resistant adhesive sheet 130 is present between the electric resistance pattern 144 and the first high frequency electrode 110, and thus heat generated by the electric resistance pattern 144 is efficiently transferred to the first high frequency electrode 110.

In order to efficiently transfer heat generated by the electrothermal conversion element 140 to the first high frequency electrode 110, it is preferable that the cover member 150 and the first holding member main body 326 around the same have lower thermal conductivity than the first high frequency electrode 110 or the highly heat-conductive and heat-resistant adhesive sheet 130. The cover member 150 and the first holding member main body 326 have low thermal conductivity so that loss of the heat generated by the electrothermal conversion element 140 is decreased.

The first electrode part 100 has been described above, and the second electrode part 200 is the same as the first electrode part 100.

The operations of the therapeutic treatment apparatus 300 according to the present exemplary embodiment will be described below. The operator previously operates the input part of the control device 370 to set the output conditions of the therapeutic treatment apparatus 300, such as setting power for high frequency energy output, target temperature for thermal energy output, and heating time. The therapeutic treatment apparatus 300 may be configured such that the respective values are independently set or a set of setting values is selected depending on an operation.

The holding part 320 and the shaft 340 in the energy treatment tool 310 are inserted into the abdominal cavity via the peritoneum, for example. The operator operates the operation knobs 352 to open/close the holding part 320 so that a body tissue to be treated is gripped by the first holding member 322 and the second holding member 324. At this time, the body tissue to be treated contacts on the first main surfaces of both of the first high frequency electrode 110 provided on the first holding member 322 and the second high frequency electrode 210 provided on the second holding member 324.

When the body tissue to be treated is gripped by the holding part 320, the operator operates the foot switch 380. When the foot switch 380 is tuned ON, high frequency power for preset power is supplied from the control device 370 to the first high frequency electrode 110 and the second high frequency electrode 210 via the first high frequency electrode current line 162 passing inside the cable 360. The supplied power is on the order of 20 W to 80 W, for example. Consequently, the body tissue generates heat and the tissue is cauterized. The tissue modifies and coagulates due to the cauterization.

After the control device 370 stops outputting high frequency energy, the electrothermal conversion element 140 is supplied with power such that the temperature of the first high frequency electrode 110 reaches a target temperature. Herein, the target temperature is 200°C, for example. At this time, a current flows through the electric resistance pattern 144 of the electrothermal conversion element 140 from the control device 370 via the cable 360 and the first heater current lines 164. The electric resistance pattern 144 generates heat due to the current. The heat generated by the electric resistance pattern 144 is transferred to the first high frequency electrode 110 via the highly heat-conductive and heat-resistant adhesive sheet 130. Consequently, the temperature of the first high frequency electrode 110 increases.

Similarly, the electrothermal conversion element 230 is supplied with power such that a temperature of the second high frequency electrode 210 reaches the target temperature. The electrothermal conversion element 230 in the second electrode part 200 is supplied with power from the control device 370 via the cable 360 and the second heater current lines 264 so that the temperature of the second high frequency electrode 210 increases.

The body tissue contacting with the first high frequency electrode 110 or the second high frequency electrode 210 is further cauterized and further coagulated by the heat. When the body tissue coagulates by the heating, the thermal energy stops being output. The operator finally operates the operation knobs 352 to move the cutter 345, thereby cutting the body tissue. The treatment of the body tissue is completed with the above operations.

As described above, for example, the first high frequency electrode 110 and the second high frequency electrode 210 function as a heat transfer plate configured to contact with a body tissue on a first main surface out of the first main surface and a second main surface, which are the front and back surfaces, and to transfer heat to the body tissue. For example, the electrothermal conversion element 140 functions as an electrothermal conversion element which is provided on the second main surface of the heat transfer plate, includes extension parts extending from the heat transfer plate, and forms with an electric resistance pattern for generating heat in response to an applied voltage and first lead connection parts connected to the electric resistance pattern and provided on the extension parts. For example, the fixing parts 114 function as fixing parts which are provided on ends where the electrothermal conversion element of the heat transfer plate extends, and grip the electrothermal conversion element between them and the heat transfer plate. For example, the first heater current lines 164 function as first lead wires which are electrically connected to the first lead connection parts at the extension parts and supply the electric resistance pattern with power. For example, the second lead connection part 148 functions as a second lead connection part which is formed to contact with the fixing part on a fourth main surface of the extension part when a surface opposite to the heat transfer plate is assumed as a third main surface out of the third main surface and the fourth main surface which are the front and back surfaces of the electrothermal conversion element. For example, the first high frequency electrode current line 162 functions as a second lead wire which is electrically connected to the second lead connection part at the extension part and is configured to apply a high frequency voltage to the heat transfer plate.

In the first electrode part 100 according to the present exemplary embodiment, the electrothermal conversion element 140 forming the first lead connection parts 146 and the second lead connection part 148 thereon is provided to protrude from the first high frequency electrode 110 and the cover member 150. Further, the first lead connection parts 146 are arranged on the first high frequency electrode 110 side of the substrate 142. Herein, the first high frequency electrode 110 is relatively thick among the components in the first electrode part 100. As illustrated in Fig. 14, the first heater current lines 164 are connected to the first lead connection parts 146 to extend from the electrothermal conversion element 140. Due to the fact, according to the present exemplary embodiment, the first heater current lines 164 do not contribute to the thickness of the first electrode part 100 and the first electrode part 100 is realized to be thinner (lower in its height). Similarly, the second lead connection part 148 is arranged on the cover member 150 side of the substrate 142. Herein, the cover member 150 is relatively thick among the components in the first electrode part 100. As illustrated in Fig. 14, the first high frequency electrode current line 162 is connected to the second lead connection part 148 to extend from the electrothermal conversion element 140. Due to the fact, according to the present exemplary embodiment, the first high frequency electrode current line 162 does not contribute to the thickness of the first electrode part 100 and the first electrode part 100 is realized to be thinner (lower in its height) .

In the present exemplary embodiment, the electrothermal conversion element 140 is inserted into the gap 126 between the electrode bottom 111 and the fixing parts 114 of the first high frequency electrode 110. A force is applied to the extension parts 143 of the electrothermal conversion element 140 in a direction perpendicular to the extension parts 143. The electrothermal conversion element 140 is restricted by the fixing parts 114 and thus is prevented from releasing from the electrode bottom 111.

If the fixing parts 114 are not present, the electrothermal conversion element 140 is fixed on the first high frequency electrode 110 only by an adhesive force of the highly heat-conductive and heat-resistant adhesive sheet 130. At this time, the electrothermal conversion element 140 can be released from the first high frequency electrode 110 due to the structure of the first high frequency electrode 110 and the limited adhesive performance of the highly heat-conductive and heat-resistant adhesive sheet 130. If such release is caused, the first high frequency electrode 110 cannot be uniformly heated by the electrothermal conversion element 140. In the present exemplary embodiment, the fixing parts 114 are present thereby to prevent the first high frequency electrode 110 and the electrothermal conversion element 140 from releasing from each other, which prevents a non-uniform temperature on heating.

Further, in the present exemplary embodiment, the first high frequency electrode current line 162 is not directly connected to the first high frequency electrode 110, but is connected to the first high frequency electrode 110 via the second lead connection part 148 and the fixing part 114. Therefore, a flow of heat from the first high frequency electrode 110 to the first high frequency electrode current line 162 can be restricted. Consequently, a reduction in temperature of the first high frequency electrode 110 at the connection portion with the first high frequency electrode current line 162 can be restricted, which enhances a thermal efficiency in heating a body tissue.

### [Second exemplary embodiment]

A second exemplary embodiment will be described. Herein, the differences from the first exemplary embodiment will be described, and the same parts are denoted with the same reference numerals and the description thereof will be omitted. In the present exemplary embodiment, the shapes of the first high frequency electrode 110 and the second high frequency electrode 210 are different from those of the first exemplary embodiment.

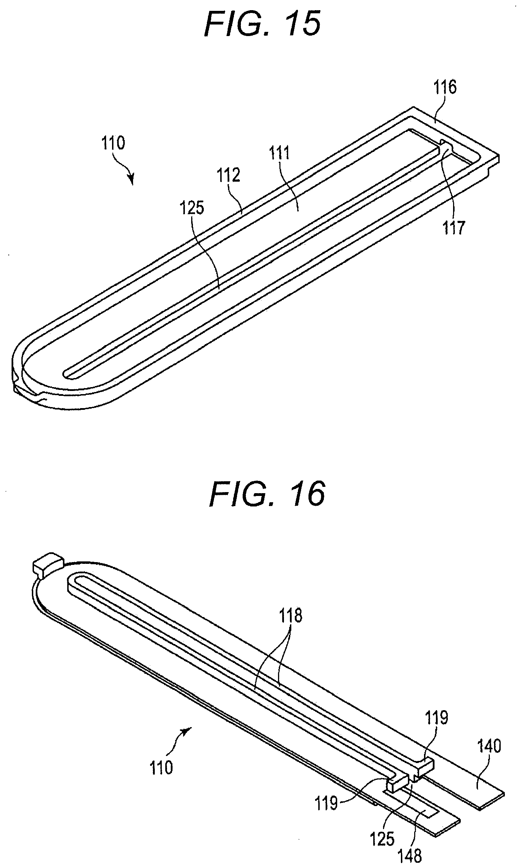

The shape of the first high frequency electrode 110 according to the present exemplary embodiment is illustrated in Fig. 15. As illustrated in Fig. 15, the first high frequency electrode 110 according to the present exemplary embodiment comprises a coupling fixing part 116 where the two fixing parts 114 symmetrically formed across the cutout 125 forming the first cutter guide groove 332 are coupled in the first exemplary embodiment. The coupling fixing part 116 is formed with a groove 117 through which the cutter 345 passes.

Other components are the same as those of the first exemplary embodiment. The present exemplary embodiment also functions like the first exemplary embodiment, and can obtain the same advantages.

The shape of the first high frequency electrode 110 may be configured such that the sidewall 112 of the first high frequency electrode 110 according to the first exemplary embodiment is not provided and an inner wall 118 is instead provided on the periphery of the cutout 125 and claw parts 119 are provided on the inner wall 118 as illustrated in Fig. 16. Further, the first high frequency electrode 110 may include both of the sidewall 112 provided on the outer periphery of the first high frequency electrode and the inner wall 118. In this case, the shape of the cover member 150 is different from that of the first exemplary embodiment, and may be changed as needed.

Other components are the same as those of the first exemplary embodiment, and the present exemplary embodiment functions like the first exemplary embodiment and can obtain the same advantages.

## Claims

1. A therapeutic treatment apparatus for heating and treating a body tissue, the apparatus comprising:
a heat transfer plate (110) having a front surface and a back surface, and being configured to contact the body tissue on its front surface to transfer heat to the body tissue;
an electrothermal conversion element (140) which includes extension parts (143) extending from the heat transfer plate (110), an electric resistance pattern (144) for generating heat in response to an applied voltage, and first lead connection parts (146) connected to the electric resistance pattern (144) and provided on the extension parts (143);
fixing parts (114) provided at ends of the heat transfer plate (110) on a side where the electrothermal conversion element (140) extends, and directed to sandwich the electrothermal conversion element (140) between the heat transfer plate (110) and the fixing parts (114); and
first lead wires (164) electrically connected to the first lead connection parts at the extension parts (143) and configured to supply the electric resistance pattern (144) with power.

2. The therapeutic treatment apparatus according to claim 1,
wherein the heat transfer plate (110) is conductive,
wherein the electrothermal conversion element (140) has a front surface and a back surface,
wherein the front surface of the electrothermal conversion element (140) is arranged opposite to the heat transfer plate (110), and
wherein the apparatus further comprises:
a second lead connection part (148) formed to contact with the fixing part on the back surface of the electrothermal conversion element (140) on the extension part (143); and
a second lead wire electrically connected to the second lead connection part (148) at the extension part (143) and configured to apply a high frequency voltage to the heat transfer plate (110).

3. The therapeutic treatment apparatus according to claim 1 or 2, further comprising:
a cover member (150) for covering the electrothermal conversion element (140) between it and the heat transfer plate (110),
wherein the cover member (150) has protrusion parts engaging with the fixing parts, and
the heat transfer part and the cover member (150) are joined with each other via the engagements between the fixing parts and the protrusion parts.

4. The therapeutic treatment apparatus according to claim 1,
wherein the electrothermal conversion element (140) comprises a front surface and a back surface,
wherein the front surface of the electrothermal conversion element (140) is arranged opposite to the heat transfer plate (110),
wherein the first lead wires (164) are arranged on the front surface of the electrothermal conversion element (140) on the extension parts (143).

5. The therapeutic treatment apparatus according to claim 2,
wherein the first lead wires (164) are arranged on the front surface of the electrothermal conversion element (140) on the extension parts (143).

6. The therapeutic treatment apparatus according to claim 1,
wherein the electrothermal conversion element (140) comprises a front surface and a back surface,
wherein the front surface is arranged opposite to the heat transfer plate (110),
wherein the electric resistance pattern (144) is provided on the front surface of the electrothermal conversion element (140) , and
the apparatus further comprises an adhesive sheet which is arranged between the heat transfer plate (110) and the electrothermal conversion element (140) and serves to thermally couple the electric resistance pattern (144) and the heat transfer plate (110).

7. The therapeutic treatment apparatus according to claim 2,
wherein the electric resistance pattern (144) is provided on the front surface of the electrothermal conversion element (140), and
the apparatus further comprises an adhesive sheet which is arranged between the heat transfer plate (110) and the electrothermal conversion element (140) and serves to thermally couple the electric resistance pattern (144) and the heat transfer plate (110).

## Patentansprüche

1. Therapeutisches Behandlungsvorrichtung zum Erwärmen und Behandeln eines Körpergewebes, wobei die Vorrichtung umfasst:
eine Wärmeübertragungsplatte (110) mit einer Vorderfläche und einer Rückfläche, die dazu konfiguriert ist, das Körpergewebe auf seiner Vorderfläche zu kontaktieren, um Wärme auf das Körpergewebe zu übertragen;
ein elektrothermisches Umwandlungselement (140), das Verlängerungsteile (143), die sich von der Wärmeübertragungsplatte (110) erstrecken, ein elektrisches Widerstandsmuster (144) zum Erzeugen von Wärme als Reaktion auf eine angelegte Spannung und erste Leitungsverbindungsteile (146), die mit dem elektrischen Widerstandsmuster (144) verbunden und an den Verlängerungsteilen (143) vorgesehen sind, umfasst;
Befestigungsteile (114), die an Enden der Wärmeübertragungsplatte (110) auf einer Seite vorgesehen sind, auf der sich das elektrothermische Umwandlungselement (140) erstreckt, und die so ausgerichtet sind, dass sie das elektrothermische Umwandlungselement (140) zwischen der Wärmeübertragungsplatte (110) und den Befestigungsteilen (114) einklemmen; und
erste Leitungskabel (164), die mit den ersten Leitungsverbindungsteilen an den Verlängerungsteilen (143) elektrisch verbunden und dazu konfiguriert sind, das elektrische Widerstandsmuster (144) mit Energie zu versorgen.

2. Therapeutische Behandlungsvorrichtung nach Anspruch 1,
wobei die Wärmeübertragungsplatte (110) leitfähig ist,
wobei das elektrothermische Umwandlungselement (140) eine Vorderfläche und eine Rückfläche aufweist,
wobei die Vorderfläche des elektrothermischen Umwandlungselements (140) der Wärmeübertragungsplatte gegenüberliegend (110) angeordnet ist, und
wobei die Vorrichtung ferner umfasst:
einen zweiten Leitungsverbindungsteil (148), der zum Kontakt mit dem Befestigungsteil auf der Rückfläche des elektrothermischen Umwandlungselements (140) auf dem Verlängerungsteil (143) ausgebildet ist; und
ein zweites Leitungskabel, das mit dem zweiten Leitungsanschlussteil (148) am Verlängerungsteil (143) elektrisch verbunden ist und dazu konfiguriert ist, eine Hochfrequenzspannung an die Wärmeübertragungsplatte (110) anzulegen.

3. Therapeutische Behandlungsvorrichtung nach Anspruch 1 oder 2, ferner umfassend:
ein Abdeckelement (150) zum Abdecken des elektrothermischen Umwandlungselements (140) zwischen sich und der Wärmeübertragungsplatte (110),
wobei das Abdeckelement (150) Vorsprungsteile aufweist, die in die Befestigungsteile eingreifen, und
der Wärmeübertragungsteil und das Abdeckelement (150) über die Eingriffe zwischen den Befestigungsteilen und den Vorsprungsteilen miteinander verbunden sind.

4. Therapeutische Behandlungsvorrichtung nach Anspruch 1,
wobei das elektrothermische Umwandlungselement (140) eine Vorderfläche und eine Rückfläche umfasst,
wobei die Vorderfläche des elektrothermischen Umwandlungselements (140) der Wärmeübertragungsplatte gegenüberliegend (110) angeordnet ist,
wobei die ersten Leitungskabel (164) auf der Vorderfläche des elektrothermischen Umwandlungselements (140) auf den Verlängerungsteilen (143) angeordnet sind.

5. Therapeutische Behandlungsvorrichtung nach Anspruch 2,
wobei die ersten Leitungskabel (164) auf der Vorderfläche des elektrothermischen Umwandlungselements (140) auf den Verlängerungsteilen (143) angeordnet sind.

6. Therapeutische Behandlungsvorrichtung nach Anspruch 1,
wobei das elektrothermische Umwandlungselement (140) eine Vorderfläche und eine Rückfläche umfasst,
wobei die Vorderfläche der Wärmeübertragungsplatte (110) gegenüberliegend angeordnet ist,
wobei das elektrische Widerstandsmuster (144) auf der Vorderflache des elektrothermischen Umwandlungselements (140) vorgesehen ist, und
die Vorrichtung ferner eine Klebefolie umfasst, die zwischen der Wärmeübertragungsplatte (110) und dem elektrothermischen Umwandlungselement (140) angeordnet ist und dazu dient, das elektrische Widerstandsmusters (144) und die Wärmeübertragungsplatte (110) thermisch zu koppeln.

7. Therapeutische Behandlungsvorrichtung nach Anspruch 2,
wobei das elektrische Widerstandsmuster (144) auf der Vorderfläche des elektrothermisches Umwandlungselements (140) vorgesehen ist und
die Vorrichtung ferner eine Klebefolie umfasst, die zwischen der Wärmeübertragungsplatte (110) und dem elektrothermischen Umwandlungselement (140) angeordnet ist und dazu dient, das elektrische Widerstandsmuster (144) und die Wärmeübertragungsplatte (110) thermisch zu koppeln.

## Revendications

1. Appareil de traitement thérapeutique pour chauffer et traiter un tissu corporel, l'appareil comprenant :
une plaque de transfert de chaleur (110) ayant une surface avant et une surface arrière, et configurée pour entrer en contact avec le tissu corporel sur sa surface avant pour transférer de la chaleur au tissu corporel ;
un élément de conversion électrothermique (140) qui comprend des parties d'extension (143) s'étendant à partir de la plaque de transfert de chaleur (110), un motif de résistance électrique (144) pour générer de la chaleur en réponse à une tension appliquée, et des premières parties de raccordement de fil (146) reliées au motif de résistance électrique (144) et prévues sur les parties d'extension (143) ;
des parties de fixation (114) prévues aux extrémités de la plaque de transfert de chaleur (110) sur un côté où l'élément de conversion électrothermique (140) s'étend, et dirigées pour prendre en sandwich l'élément de conversion électrothermique (140) entre la plaque de transfert de chaleur (110) et les parties de fixation (114) ; et
des premiers fils (164) reliés électriquement aux premières parties de raccordement de fil au niveau des parties d'extension (143) et configurés pour alimenter électriquement le motif de résistance électrique (144).

2. Appareil de traitement thérapeutique selon la revendication 1,
la plaque de transfert de chaleur (110) étant conductrice,
l'élément de conversion électrothermique (140) ayant une surface avant et une surface arrière,
la surface avant de l'élément de conversion électrothermique (140) étant disposée en regard de la plaque de transfert de chaleur (110), et
l'appareil comprenant en outre :
une seconde partie de raccordement de fil (148) formée pour être en contact avec la partie de fixation sur la surface arrière de l'élément de conversion électrothermique (140) sur la partie d'extension (143) ; et
un second fil relié électriquement à la seconde partie de raccordement de fil (148) au niveau de la partie d'extension (143) et configuré pour appliquer une tension à haute fréquence à la plaque de transfert de chaleur (110).

3. Appareil de traitement thérapeutique selon la revendication 1 ou 2, comprenant en outre :
un élément de recouvrement (150) pour recouvrir l'élément de conversion électrothermique (140) entre celui-ci et la plaque de transfert de chaleur (110),
l'élément de recouvrement (150) ayant des parties en saillie s'engageant avec les parties de fixation, et
la partie de transfert de chaleur et l'élément de recouvrement (150) étant assemblés l'un à l'autre par l'intermédiaire des engagements entre les parties de fixation et les parties en saillie.

4. Appareil de traitement thérapeutique selon la revendication 1,
l'élément de conversion électrothermique (140) comprenant une surface avant et une surface arrière,
la surface avant de l'élément de conversion électrothermique (140) étant disposée en regard de la plaque de transfert de chaleur (110),
les premiers fils (164) étant disposés sur la surface avant de l'élément de conversion électrothermique (140) sur les parties d'extension (143).

5. Appareil de traitement thérapeutique selon la revendication 2,
les premiers fils (164) étant disposés sur la surface avant de l'élément de conversion électrothermique (140) sur les parties d'extension (143).

6. Appareil de traitement thérapeutique selon la revendication 1,
l'élément de conversion électrothermique (140) comprenant une surface avant et une surface arrière,
la surface avant étant disposée en regard de la plaque de transfert de chaleur (110),
le motif de résistance électrique (144) étant prévu sur la surface avant de l'élément de conversion électrothermique (140), et
l'appareil comprenant en outre une feuille adhésive qui est disposée entre la plaque de transfert de chaleur (110) et l'élément de conversion électrothermique (140) et sert à coupler thermiquement le motif de résistance électrique (144) et la plaque de transfert de chaleur (110).

7. Appareil de traitement thérapeutique selon la revendication 1,
le motif de résistance électrique (144) étant prévu sur la surface avant de l'élément de conversion électrothermique (140), et
l'appareil comprenant en outre une feuille adhésive qui est disposée entre la plaque de transfert de chaleur (110) et l'élément de conversion électrothermique (140) et sert à coupler thermiquement le motif de résistance électrique (144) et la plaque de transfert de chaleur (110).
